# EUROPEAN PATENT APPLICATION

(11) **EP 4 748 392 A1**
(43) Date of publication of application: **27.05.2026**
(21) Application number: 24214225.5
(22) Date of filing: 20.11.2024
(51) Int. Cl.: A61K 39/09, A61K 9/00, C07K 14/00

(54) **VACCINE FORMULATION**

(71) Applicant: MinervaX ApS, 2000 Frederiksberg (DK)
(72) Inventor: FISCHER, Per, 2000 FREDERIKSBERG (DK); KANTSØ, Bjørn, 4300 HOLBÆK (DK); BOMHOLT, Julie, 2920 CHARLOTTENLUND (DK)
(74) Representative: Öberg & Sjöberg AB

(57) **Abstract**

The technology proposed herein concerns a vaccine formulation comprising a) an antigen comprising an amino acid sequence having at least 90% sequence identity with the amino acid sequence of the N-terminal region of a first group B *Streptococcus* surface protein, and b) a buffer having a pH of 6 to 8 and comprising: a. aluminum hydroxide gel particles, and b. a buffer system comprising: i. phosphate, and optionally also a salt, or ii. histidine, and optionally also a sugar. The technology proposed herein further concerns a method of producing a vaccine formulation as well as a vaccine formulation for use in a method of reducing, preventing and/or treating a GBS infection.

## Description

### TECHNICAL FIELD

The technology proposed herein relates to the fields of microbiology and vaccine technology and concerns the development of a vaccine formulation for conferring immunity to group B Streptococcus infections.

### BACKGROUND

Group B Streptococcus (*Streptococcus agalactiae*) (GBS) is the major cause of invasive bacterial infections, including meningitis, in the neonatal period. In the United States alone, there are now about 5000 cases per year of invasive disease caused by this bacterium. These infections have an overall mortality of about 10%, and many of the infants that survive have permanent neurological sequelae. In view of this, a large effort has been made to find methods of prevention and treatment and to analyze the mechanisms by which GBS cause infections.

GBS can also cause mastitis in cows, a bovine disease that is of considerable economical importance. Development of a vaccine against GBS infections is therefore of interest also in veterinary medicine.

About 20 % of all women are vaginal carriers of GBS, and vertical transmission from the maternal genital tract is probably the most common source of infection in neonatal disease caused by this bacterium. However, only about 1 % of the infants that are colonized by the GBS at birth are afflicted by serious infection. Other factors than exposure to the bacterium during birth must therefore contribute to the development of neonatal disease.

Group B streptococcal strains are divided into nine serotypes (la, lb, and II-VIII) based on the structure of the polysaccharide capsule. The four "classical" serotypes la, Ib, II, and III occur in roughly equal proportions among strains in the normal flora, but type III is the clinically most important serotype, in particular because it causes most cases of meningitis. Because the capsule is a known virulence factor, it has been studied in considerable detail, in particular in type III strains. Efforts have been made to develop a vaccine, in which the type III polysaccharide capsule would be an essential component. WO2008127179 inter alia describes a fusion protein comprising a first N-terminal region fragment from the group B Streptococcus surface Rib protein fused to a second N-terminal region fragment from the group B Streptococcus surface Rib protein.

WO2017068112 inter alia describes a fusion protein comprising a first N-terminal region fragment from the group B Streptococcus surface Alp1 protein fused to a second N-terminal region fragment from the group B Streptococcus surface Alp2 protein.

Despite the advances in the progress towards a vaccine suitable for prevention of GBS disease, there is still a need for efficient vaccine formulations capable of delivering antigens so as to invoke an effective immune response for prevention and treatment of GBS infections.

### OBJECTS

Accordingly, it is a primary objective of the technology proposed herein to provide a vaccine formulation for reducing, preventing and/or treating a GBS infection.

Another objective of the technology proposed herein is to provide a method of producing the vaccine formulation.

A further objective of the technology proposed herein is to provide a vaccine formulation for use in a method of reducing, preventing and/or treating a GBS infection, as well a method of reducing, preventing and/or treating a GBS infection by administering a vaccine formulation.

### SUMMARY

At least one of the above objects, or at least one of further objects which will be clear from the following description, are according to first and second corresponding aspects of the technology proposed herein achieved by a vaccine formulation comprising:
a) an antigen comprising an amino acid sequence having at least 90% sequence identity with the amino acid sequence of the N-terminal region of a group B *Streptococcus* surface protein, and
b) a buffer having a pH of 6 to 8 and comprising:
   a. aluminum hydroxide gel particles, and
   b. a buffer system comprising:
      i. phosphate, and optionally also a salt, or
      ii. histidine, and optionally also a sugar,
and
a method of producing a vaccine formulation comprising the steps of:
i. providing an antigen comprising an amino acid sequence having at least 90% sequence identity with the amino acid sequence of the N-terminal region of a group B *Streptococcus* surface protein,
ii. providing a buffer having a pH of 6 to 8 and comprising:
   a. aluminum hydroxide gel particles, and
   b. a buffer system comprising:
      i. phosphate, and optionally also a salt, or
      ii. histidine, and optionally also a sugar, and
iii. mixing the antigen with the buffer.

The technology proposed herein is thus at least in part based on the recognition that an antigen comprising an amino acid sequence having at least 90% sequence identity with the amino acid sequence of the N-terminal region of a first group B *Streptococcus* surface protein is especially well formulated, as regards low desorption and storage stability, when provided in the buffer as specified and as evaluated in the examples.

Corresponding third and further aspects of the technology proposed herein concern the vaccine formulation according to the first aspect of the technology proposed herein for use in a method of a reducing, preventing and/or treating a GBS infection, as well a method of reducing, preventing and/or treating a GBS infection by administering the vaccine formulation according to the first aspect of the technology proposed herein.

### BRIEF DESCRIPTION OF THE FIGURES

- Figs. 1A-1D: show desorption (concentration of free fusion proteins) of the first and second fusion proteins from the candidate vaccine formulations F(DP)1-F(DP)7 versus time of up to 12 months at 5°C and 25° C.
- Figs. 2A-2L: show measured fluorescence intensity of Trp and Tyr amino acid residues in the fusion proteins as the temperatures of the candidate vaccine formulations F(DP)1-F(DP)7 are ramped.
- Figs. 3A-3F: show inhibition Elisa assays curves for the vaccine formulation candidates having been stored at different times and temperatures compared to a reference material (freshly prepared fusion proteins).

### DETAILED DESCRIPTION

The first and second corresponding aspects of the technology proposed herein concern a vaccine formulation comprising:
a) an antigen comprising an amino acid sequence having at least 90% sequence identity with the amino acid sequence of the N-terminal region of a group B *Streptococcus* surface protein, and
b) a buffer having a pH of 6 to 8 and comprising:
   a. aluminum hydroxide gel particles, and
   b. a buffer system comprising:
      i. phosphate, and optionally also a salt, or
      ii. histidine, and optionally also a sugar,
and
a method of producing a vaccine formulation comprising the steps of:
i. providing an antigen comprising an amino acid sequence having at least 90% sequence identity with the amino acid sequence of the N-terminal region of a group B *Streptococcus* surface protein,
ii. providing a buffer having a pH of 6 to 8 and comprising:
   a. aluminum hydroxide gel particles, and
   b. a buffer system comprising:
      i. phosphate, and optionally also a salt, or
      ii. histidine, and optionally also a sugar, and
iii. mixing the antigen with the buffer.

The technology proposed herein is thus at least in part based on the recognition that an antigen comprising an amino acid sequence having at least 90% sequence identity with the amino acid sequence of the N-terminal region of a first group B *Streptococcus* surface protein is especially well formulated, as regards low desorption and storage stability, when provided in the buffer as specified and as evaluated in the examples.

Specifically, as shown in the examples, the antigen has low desorption from the aluminum hydroxide gel particles, maintains its intended folding, i.e. undergoes no or little unfolding, and provides similar immunological response to antibodies as compared toa non-stored reference antigen, despite having been stored for long times at advantageous storage temperatures.

The vaccine formulation may alternatively be considered an immunogenic composition. In the context of the technology proposed herein a vaccine composition is a composition that is capable of invoking an at least partial protection against a pathogen. Partial protection may encompass a reduction or prevention of one or more of the pathogen entering a subjects body or cells, the pathogen causing a disease or symptoms to the subject, or a reduction of the extent of infection by the pathogen and/or a reduction of the duration of disease or symptoms caused by the pathogen. In the context of the technology proposed herein the pathogen is preferably a bacteria, more preferably a group B Streptococcus (GBS) bacteria. A vaccine formulation may in particular invoke antibodies against the pathogen or against one or more components of the pathogen, in particular surface proteins, parts of surface proteins, when administered to a subject having an immune system.

The vaccine formulation is preferably suitable for administration by injection, in particular intramuscular injection, e.g. suitable for parenteral administration.

The antigen is a molecule that can bind to an antibody or T-cell receptor, The antigen can comprise proteins, peptides (amino acid chains), and/or polysaccharides (chains of simple sugars).

The antigen may alternatively be considered an immunogenic compound. An immunogenic compound, like an antigen, is a molecule that can bind to an antibody or T-cell receptor. The antigen may in particular trigger an immune response when administered to a subject having an immune system.

The antigen comprises an amino acid sequence having at least 90% sequence identity with the amino acid sequence of the N-terminal region of a group B Streptococcus surface protein.

According to the technology proposed herein, sequence identity is calculated in the following manner. By an amino or nucleic acid sequence having a sequence with at least, for example 95%, identity to a reference sequence, is intended that the sequence is identical to the reference sequence except that the sequence may include up to 5 amino or nucleic acid differences per each 100 amino or nucleic acids of the reference sequence. In other words, to obtain an amino or nucleic acid sequence having a sequence identity of at least 95%, up to 5% of the amino or nucleic acids in the reference sequence may be deleted or substituted with another amino or nucleic acid, or a number of amino or nucleic acids up to 5% of the total number of amino or nucleic acids in the reference sequence may be inserted into the reference sequence. If the two sequences to be compared are not of equal length, they must be aligned to best possible fit, and the sequence identity is calculated over the length of the reference sequence. These amino or nucleic acid changes of the reference sequence may occur at the amino and/or carboxy terminal positions for an amino acid sequence or at the 5' and/or 3' end for a nucleic acid sequence of a reference amino or nucleic acid sequence or anywhere between those terminal positions, interspersed either individually within the reference sequence or in one or more contiguous groups within the reference sequence.

The amino acid sequence may alternatively be considered a polypeptide or protein.

Preferably, the antigen comprises an amino acid sequence having at least 91%, preferably at least 92%, such as at least 93% or at least 94% sequence identity, more preferably at least 95% such as at least 96%, at least 97% or at least 98% most preferably at least 99% or 100%, sequence identity with the amino acid sequence of the N-terminal region of a group B Streptococcus surface protein.

Group B Streptococcus surface proteins include Rib, AlpC, Alp1, Alp2, Alp3 and Alp4. The Rib protein, also referred to in this specification as Rib, is a surface protein for example described in WO 9421685. The denotation "Rib" refers to: Resistance to proteases, immunity, and group B. The Rib protein was first isolated from a group B streptococcal strain of serotype III as a distinct 95 kDa protein. Protein Rib is expressed by almost all group B streptococcal strains of the clinically important serotype III, which cause most cases of meningitis, and by some strains of other serotypes such as II. Moreover, Rib is expressed by all strains of a hypervirulent clone of type III. The amino acid sequence of the N-terminal of Rib is given by SEQ ID NO: 1.

The AlpC protein, also known as alpha protein, is a group B Streptococcus surface protein. The amino acid sequence of the N-terminal of AlpC is given by SEQ ID NO: 2. The Alp1 protein is a group B streptococcal alpha-protein-like protein. The amino acid sequence of the N-terminal of Alp1 is given by SEQ ID NO: 4.

The Alp2 protein is another alpha-protein-like-protein first identified in a serotype V strain. Like the other members of the family, the Alp2 protein has an N-terminal domain and several repeated domains towards the C-terminus. The amino acid sequence of the N-terminal of Alp2 is given by SEQ ID NO: 5.

The Alp3 protein is yet another alpha-protein-like-protein. It is very similar to the R28 protein also found in *S. pyrogenes.* Alp3 has an N-terminal domain which is identical to that of Alp2. Accordingly, the amino acid sequence of the N-terminal of Alp3 is also given by SEQ ID NO: 5.

The Alp4 protein is an alpha-protein-like-protein. It has a distinct N-terminal domain, and repeat regions towards the C-terminus. The amino acid sequence of the N-terminal of Alp4 is given by SEQ ID NO: 7.

The antigen with the amino acid sequence having at least 90% sequence identity with the amino acid sequence of the N-terminal region of a group B Streptococcus surface protein may provided on its own as the antigen. Alternatively, the antigen may comprise two or more such amino acid sequences.

The amino acid sequence may be modified by glycosylation, amidation, carboxylation or phosphorylation.

The one or more amino acid sequences may carry one or more capsular polysaccharides. In other words, the one or more amino acid sequences may form a complex with the one or more capsular polysaccharides. The capsular polysaccharide may be a Group B Streptococcus capsular polysaccharide. The capsular polysaccharide may be conjugated to the amino acid sequence by a covalent or non-covalent bond or linkage. Any linkage known to the skilled person may be used to link a capsular polysaccharide and an amino acid sequence. In particular, the capsular polysaccharide may be coupled or conjugated to the amino acid sequence by a spacer molecule, such as adipic acid. This spacer molecule can be used to facilitate the coupling or conjugation of the amino acid sequence to polysaccharide.

Another non-limiting example of coupling or conjugation of an amino acid sequence to a capsular polysaccharide is non-covalent conjugation using biotin and a biotin-binding moiety. The capsular polysaccharide may be biotinylated and the amino acid sequence may comprise a biotin-binding moiety or vice versa. The biotin-binding moiety may be rhizavidin. Another non-limiting example of non-covalent coupling or conjugation is the use of a sialic acid binding moiety. The capsular polysaccharide may comprise the sialic acid and the amino acid sequence may comprise a sialic acid-binding moiety or vice versa. The sialic acid-binding moiety may be SBD1, SBD2, SBD3, SBD4, NanH, NanH2, or VcNanH. Another non-limiting example of non-covalent coupling or conjugation is the use of biotin and streptavidin, whereby the capsular polysaccharide comprises the biotin and the amino acid sequence comprises streptavidin or vice versa. Other pairs of binding moieties are readily useable.

The capsular polysaccharide is preferably a bacterial polysaccharide, more preferably a group B Streptococcus polysaccharide.

The capsular polysaccharide may be serotype specific and selected from group consisting of Group B Streptococcus serotypes la, Ib, II, III, IV, V, VI, VII, VIII, IX and X.

By polysaccharide is meant any linear or branched polymer consisting of monosaccharide residues, usually linked by glycosidic linkages, and thus includes oligosaccharides. Preferably, the polysaccharide will contain between 2 and 50 monosaccharide units, more preferably between 6 and 30 monosaccharide units. A particularly preferred method of coupling polysaccharide and the amino acid sequence is by reductive amination. Other methods include: activation of the polysaccharide with cyanogen bromide followed by reaction with adipic acid dihydrazide (spacer) and by conjugation to carboxide groups of carrier amino acid sequences or protein using soluble carbodiimides; functionalisation of the carrier amino acid sequence or protein with adipic acid dihydrazide followed by coupling to cyanogen bromide activated polysaccharides; chemical modification of both the carrier amino acid sequence and the polysaccharide followed by their coupling.

The polysaccharide molecule may be coupled to the amino acid sequence by a spacer molecule, such as adipic acid. This spacer molecule can be used to facilitate the coupling of amino acid sequence to polysaccharide. A spacer may also be denoted a linker.

After the coupling reaction has been performed, the immunogenic complex or conjugate may be purified by diafiltration or other known methods to remove unreacted amino acid sequence or polysaccharide components.

Multiple capsular polysaccharides may be coupled or conjugated to the same amino acid sequence as described above.

The amino acid sequence used in an immunogenic complex or conjugate preferably comprises or consists of an amino acid sequence having at least 80%, such as at least 90% more preferably at least 98% or at least 99% sequence identity to one of SEQ ID NOs 1, 2, 4, 5 or 7. Preferably the amino acid sequence used in an immunogenic complex or conjugate has a length of from 172 amino acids to 178 amino acids when having at least 80% sequence identity to SEQ ID NO: 1, a length of from 167 amino acids to 173 amino acids when having at least 80% sequence identity to SEQ ID NO: 2, and the same length as SEQ ID NO: 5 when having at least 80% sequence identity to SEQ ID NO: 5.

The buffer having a pH of 6 to 8 is preferably an aqueous buffer. In other words, the buffer is preferably prepared by adding various substances to water (H₂O). The buffer preferably has a pH of 6 to 6.4 or 7 to 7.4, more preferably 6.1 to 6.3 or 7.1 to 7.3. Alternatively, the buffer has a pH of 6.1 to 7.3, such as 6.2 to 7.2.

The aluminum hydroxide gel particles are also known as Alhydrogel^{®} or AIOH. Alhydrogel^{®} is a semi-crystalline form of aluminium oxyhydroxide (AH). The aluminum hydroxide gel particles are used as an adjuvant and also binds the antigen. The aluminum hydroxide gel particles are positively charged at a neutral pH which allows them to bind, e.g. adsorb, the antigen which typically is negatively charged as it contains the amino acid sequence.

The buffer system is used to regulate the pH of the buffer. In other words, the buffer system may be considered to be configured to cause the buffer to have a pH of 6 to 8. The buffer system comprises a weak acid and it conjugate base.

The buffer system preferably comprises phosphate, and optionally also a salt. The buffer may for example comprise sodium phosphates, such as mono/di/tri-sodium phosphate or mono/di/tri-sodium di/triphosphate, preferably disodium hydrogen phosphate, in particular disodium hydrogen phosphate dihydrate (Na₂HPO₄ · 2H₂O), and/or potassium phosphates such as mono/di/tripotassium phosphate, preferably monopotassium phosphate, in particular monopotassium dihydrogen phosphate (KH₂PO₄).

Two different phosphates may be combined. Preferably a sodium phosphate is combined with a potassium phosphate. Most preferably disodium hydrogen phosphate dihydrate is combined with monopotassium dihydrogen phosphate.

The total concentration of phosphate may typically be 1-10 mM, preferably 1-5 mM, such as 1-3 mM, more preferably 1.5 to 2.5 mM, most preferably 2 mM.

In a combination the concentration of phosphate refers to the combined concentration of the respective phosphates. Accordingly, for a combination of disodium hydrogen phosphate dihydrate and monopotassium dihydrogen phosphate the combined concentration of the respective phosphate is preferably 1-10 mM, preferably 1-5 mM, such as 1-3 mM, more preferably 1.5 to 2.5 mM, most preferably 2 mM.

In the combination of two phosphates the molar ratio between the phosphates may be from 2:1 to 4:1, preferably from 2.5:1 to 3.5:1, mot preferably 3:1.

Accordingly, for a combination of disodium hydrogen phosphate dihydrate and monopotassium dihydrogen phosphate the ratio of the former to the latter may be from 2:1 to 4:1, preferably from 2.5:1 to 3.5:1, mot preferably 3:1. For such a buffer, with the ratio 3:1 and comprising 2mM phosphate, 1.5 mM of the 2 mM concentration of phosphate is due to the Na₂HPO₄ · 2H₂O and the remaining 0.5 mM concentration of phosphate is due to the KH₂PO₄.

The salt may preferably comprise NaCl and/or Cl. The total concentration of salt may be 50-400 mM, preferably 100 -350 mM, more preferably 100-200 mM, such as 125-175 mM or 145-155 mM such as 150 mM.

As seen in the examples, the addition of the salt decreases desorption of the antigen from the aluminum hydroxide gel particles.

The buffer may for example comprise Phosphate-buffered saline (PBS).

The buffer system may alternatively comprise histidine, and optionally also a sugar. The histidine is preferably L-histidine. Histidine has pKas at 1.54, 6.07, and 9.34, and provides a buffering effect near these pKAs.

The sugar may for example be sorbitol or sucrose, preferably sorbitol.

The concentration of histidine may preferably be 1-20 mM. The concentration of the sugar may preferably be 100-500 mM, more preferably 200-400 mM, such as 300 mM.

The method of producing a vaccine formulation is preferably a method of producing the vaccine formulation according to the first aspect of the technology proposed herein.

The steps i-iii are preferably performed in order, but may be performed in any order provided that step (iii) is performed after steps i and ii.

The step i of providing an antigen preferably encompasses providing the antigen in an antigen containing solution.

The step ii of providing a buffer preferably encompasses providing a finished buffer, but may alternatively encompass mixing the components of the buffer to prepare the finished buffer.

The step iii of mixing the antigen with the buffer may be performed using any suitable mixer.

Preferably the antigen comprises a fusion protein comprising or consisting of a first amino acid sequence having at least 90% sequence identity with the amino acid sequence of the N-terminal region of a first group B *Streptococcus* surface protein, said first amino acid sequence being fused, optionally via linker, to a second amino acid sequence having at least 90% sequence identity with the amino acid sequence of the N-terminal region of a second group B *Streptococcus* surface protein.

The first and second group B *Streptococcus* surface proteins are preferably different. Such an antigen may provide an immunogenic effect, and hence serve in a vaccine formulation having effect against two different GBS surface proteins, thus providing a broader coverage since not all GBS serotypes express all GBS surface proteins.

The first and second amino acid sequences may preferably be separated by a linker. This linker is typically 1-20 amino acids long, preferably 1 to 10 amino acids, preferably 2 to 5 amino acids, more preferably 2 amino acids, and most preferably the linker consists of the amino acid sequence EF. The function of the linker is to join the first and second group amino acid sequences. The linker is typically a linker that is not cleavable.

The fusion protein is preferably an immunogenic fusion protein, i.e. a fusion protein able to elicit a protective immunity against group B Streptococcus. The fusion protein preferably consists of said first and second group B Streptococcus surface proteins and optionally a linker.

Preferably the first group B *Streptococcus* surface protein is selected from the group consisting of Rib protein and Alp1 protein, and wherein the second group B *Streptococcus* surface protein is selected from the group consisting of AlpC protein, Alp2 protein, Alp3 protein, and Alp4 protein.

This is advantageous because the corresponding combinations of N terminals of surface proteins, in particular the combinations RibN-AlpCN and Alp1/Alp2/3N each provide broad immunogenic effect against a large variety of GBS serotypes.

Further combinations of N terminals of surface proteins include: RibN-Alp2/3N, RibN-Alp4N, Alp1N-Alp4N,

It is further contemplated that the first group B *Streptococcus* surface protein may be selected from the group consisting of Rib protein and AlpC protein, and the second group B *Streptococcus* surface protein is selected from the group consisting of Alp1 protein, Alp2 protein, Alp3 protein, and Alp4.

Such combinations of N terminals of surface proteins include: RibN-Alp1N, AlpCN-Alp1N, AlpCN-Alp2/3N, and AlpCN-Alp4N.

Preferably the first group B *Streptococcus* surface protein is Rib protein, and the second group B *Streptococcus* surface protein is AlpC protein. This results in a first fusion protein which comprises or consists of a first amino acid sequence having at least 90% sequence identity with the amino acid sequence of the N-terminal region of Rib, said first amino acid sequence being fused, optionally via linker, to a second amino acid sequence having at least 90% sequence identity with the amino acid sequence of the N-terminal region of AlpC

This first fusion protein preferably consists of:
- a first amino acid sequence part consisting of 170 to 178 amino acids, preferably 174 to 175 amino acids, and having at least 90% sequence identity with the amino acid sequence shown in SEQ ID NO: 1;
- a second amino acid sequence part consisting of 165 to 174 amino acids, preferably 169 to 170 amino acids, and having at least 90% sequence identity with the amino acid sequence shown in SEQ ID NO: 2; and optionally:
- a linker amino acid sequence part consisting of 1 to 20 amino acids and separating the first amino acid sequence part from the second amino acid part,

and wherein the first fusion protein preferably consists of 335 to 372 amino acids,
preferably 343 to 353 amino acids, more preferably 343 to 347 amino acids,
and wherein preferably the first fusion protein has an amino acid sequence having at least 90%, at least 92%, at least 94%, at least 96%, at least 98%, at least 99%, or 100% identity to SEQ ID NO: 3.

Such a fusion protein gives a broad immunogenic effect and/or protection against GBS bacteria expressing the surface proteins Rib and/or AlpC.

Further, details of such a recombinant fusion protein can be found in WO2022/207657. SEQ ID NO: 3 is the sequence for a fusion protein between the N-terminal regions of Rib and AlpC with an EF linker, herein also denoted GBS-NN.

GBS-NN is a non-glycosylated protein (no other post-translational modifications) with a molecular weight of 38.2 kDa and a pl of 4.8.

Alternatively, the first group B *Streptococcus* surface protein is Alp1 protein, and the second group B *Streptococcus* surface protein is Alp2 protein. This results in a second fusion protein which comprises or consists of a first amino acid sequence having at least 90% sequence identity with the amino acid sequence of the N-terminal region of Alp1, said first amino acid sequence being fused, optionally via linker, to a second amino acid sequence having at least 90% sequence identity with the amino acid sequence of the N-terminal region of Alp2.

This second fusion protein preferably consists of:
- a first amino acid sequence part consisting of 168 to 177 amino acids, preferably 172 to 173 amino acids, and having at least 90% sequence identity with the amino acid sequence shown in SEQ ID NO: 4,
- a second amino acid sequence part consisting of 168 to 178 amino acids, preferably 172 to 173 amino acids, and having at least 90% sequence identity with the amino acid sequence shown in SEQ ID NO: 5, and optionally,
- a linker amino acid sequence part consisting of 1 to 20 amino acids and separating the first amino acid sequence part from the second amino acid part, and wherein the second fusion protein preferably consists of 335 to 372 amino acids, preferably 343 to 353 amino acids, more preferably 343 to 347 amino acids,
and wherein preferably the second fusion protein has an amino acid sequence having at least 90%, at least 92%, at least 94%, at least 96%, at least 98%, at least 99%, or 100% identity to SEQ ID NO: 6.

Such a fusion protein gives a broad immunogenic effect and/or protection against GBS bacteria expressing the surface proteins Alp1 and/or Alp2 and/or Alp3.

SEQ ID NO: 6 is the sequence for a fusion protein between the N-terminal regions of Alp1 and Alp2 with an EF linker, herein denoted GBS-NN2.

GBS-NN2 is a non-glycosylated protein (no other post-translational modifications) with a molecular weight of 38.1 kDa and a pl of 4.2.

The antigen may comprise two fusion proteins as described above. In that case the group B *Streptococcus* surface proteins, to which the respective amino acid sequences have at least 90% sequence identity to, are preferably selected to all be different.

Accordingly, it is then preferred that the two fusion proteins are the first and second fusion proteins as described above. In other words, it is preferred when the antigen comprises the combination of the first fusion protein and the second fusion protein. Such an antigen gives a broad immunogenic effect and/or protection against GBS bacteria expressing any of the surface proteins Rib, AlpC, Alp1, Alp2, and Alp3.

Accordingly, the antigen preferably comprises or consists of:
- a first fusion protein as defined above making reference to the group B *Streptococcus* surface proteins Rib and AlpC, or more preferably by making reference to SEQ ID Nos 1 and 2, most preferably by making reference to SEQ ID NO: 3, and
- a second fusion protein as defined above by making reference to the group B *Streptococcus* surface proteins Alp1 and Alp2, or more preferably by making reference to SEQ ID Nos: 4 and 5, most preferably by making reference to SEQ ID NO: 6.

In other words, the antigen preferably comprises or consists of
- a first fusion protein comprising or consisting of a first amino acid sequence having at least 90% sequence identity with the amino acid sequence of the N-terminal region of Rib, said first amino acid sequence being fused, optionally via linker, to a second amino acid sequence having at least 90% sequence identity with the amino acid sequence of the N-terminal region of AlpC,
   wherein the first fusion protein preferably consists of:
      - a first amino acid sequence part consisting of 170 to 178 amino acids, preferably 174 to 175 amino acids, and having at least 90% sequence identity with the amino acid sequence shown in SEQ ID NO: 1;
      - a second amino acid sequence part consisting of 165 to 174 amino acids, preferably 169 to 170 amino acids, and having at least 90% sequence identity with the amino acid sequence shown in SEQ ID NO: 2; and optionally:
      - a linker amino acid sequence part consisting of 1 to 20 amino acids and separating the first amino acid sequence part from the second amino acid part, and wherein the first fusion protein preferably consists of 335 to 372 amino acids,
   preferably 343 to 353 amino acids, more preferably 343 to 347 amino acids, and wherein more preferably the first fusion protein has an amino acid sequence having at least 90%, at least 92%, at least 94%, at least 96%, at least 98%, at least 99%, or 100% identity to SEQ ID NO: 3, and
- a second fusion protein which comprises or consists of a first amino acid sequence having at least 90% sequence identity with the amino acid sequence of the N-terminal region of Alp1, said first amino acid sequence being fused, optionally via linker, to a second amino acid sequence having at least 90% sequence identity with the amino acid sequence of the N-terminal region of Alp2,
   wherein the second fusion protein preferably consists of:
   - a first amino acid sequence part consisting of 168 to 177 amino acids, preferably 172 to 173 amino acids, and having at least 90% sequence identity with the amino acid sequence shown in SEQ ID NO: 4,
   - a second amino acid sequence part consisting of 168 to 178 amino acids, preferably 172 to 173 amino acids, and having at least 90% sequence identity with the amino acid sequence shown in SEQ ID NO: 5, and optionally,
   - a linker amino acid sequence part consisting of 1 to 20 amino acids and separating the first amino acid sequence part from the second amino acid part, and wherein the second fusion protein preferably consists of 335 to 372 amino acids, preferably 343 to 353 amino acids, more preferably 343 to 347 amino acids, and wherein more preferably the second fusion protein has an amino acid sequence having at least 90%, at least 92%, at least 94%, at least 96%, at least 98%, at least 99%, or 100% identity to SEQ ID NO: 6.

More preferably, the antigen comprises, or preferably consists, of
- a first fusion protein having at least 90%, at least 92%, at least 94%, at least 96%, at least 98%, at least 99%, or 100% identity to SEQ ID NO: 3 and
- a second fusion protein having at least 90%, at least 92%, at least 94%, at least 96%, at least 98%, at least 99%, or 100% identity to SEQ ID NO: 6.

Most preferably, the antigen consists of:
- a first fusion protein having the amino acid sequence of SEQ ID NO: 3, and
- as second fusion protein having the amino acid sequence of SEQ ID NO: 6.

When the antigen comprises or consists of:
- a first fusion protein as discussed above, and
- a second fusion protein as discussed above,
then it is preferred that:
- the concentration of the first fusion protein, in the vaccine formulation, is from 0.05 to 0.15 mg/ml, preferably 0.75 to 0.125 mg/ml, more preferably 0.09 to 0.11 mg/ml, such as 0.1, mg/ml, and
- the concentration of the second fusion protein, in the vaccine formulation, is from 0.05 to 0.15 mg/ml, preferably 0.75 to 0.125 mg/ml, more preferably 0.09 to 0.11 mg/ml, such as 0.1 mg/ml.

Generally, the buffer preferably comprises 0.5 - 1.5 mg/ml, preferably 0.75-1.25 mg/ml, more preferably 0.9-1.1. mg/ml, such as 1 mg/ml, of aluminum hydroxide gel particles. These concentrations of aluminum hydroxide gel particles are suitable to adsorb and bind the antigen.

Generally, the buffer system preferably comprises:
i. 1-3 mM, preferably 1.5 to 2.5 mM, more preferably 2 mM phosphate, and preferably also 100-200 mM, preferably 125-175 mM, more preferably 150 mM NaCl, or
ii. 5-15, preferably 8-12, more preferably 10 mM histidine, and preferably also 200-400 mM, preferably 250-350 mM, more preferably 300 mM sorbitol.

As shown by the examples, such buffer systems provide good stability to the antigen and low desorption.

Most preferably, the vaccine formulation comprises:
a) an antigen consisting of 0.1 mg/ml of a fusion protein having the amino acid sequence of SEQ ID NO: 3 and 0.1 mg/ml of a fusion protein having the amino acid sequence of SEQ ID NO: 6, and
b) a buffer having a pH of 7.2 and comprising:
   a. 1 mg/ml aluminum hydroxide gel particles,
   b. 2 mM Na/K phosphate, and
   c. 150 mM NaCl.

As shown by the examples, this vaccine formulation provides the overall best results. Na/K phosphate refers to a combination of a sodium phosphate and a potassium phosphate.

The 2 mM Na/K phosphate preferably comprises Na₂HPO₄ . 2H₂O and KH₂PO₄. The molar ratio of Na₂HPO₄ · 2H₂O to KH₂PO₄ preferably from 2:1 to 4:1, more preferably from 2.5:1 to 3.5:1, most preferably 3:1.

The vaccine formulation most preferably comprises or consists of the components listed in table 7 further below.

Corresponding third and fourth aspects of the technology proposed herein concern the vaccine formulation according to the first aspect of the technology proposed herein for use in a method of a reducing, preventing and/or treating a GBS infection, as well a method of reducing, preventing and/or treating a GBS infection by administering the vaccine formulation according to the first aspect of the technology proposed herein.

Preferably, the vaccine formulation is administered twice with an interval of 7-80, preferably 40-70, more preferably 50-60 such as 58-60, most preferably 57 days. Preferably the vaccine formulation is administered intramuscular (IM).

Preferably the vaccine formulation is administered to a subject, wherein the subject preferably is a female subject, wherein the subject preferably is a female subject carrying an unborn offspring.

LIST OF SEQUENCES (methionine in the N-terminal end is optional):
SEQ ID NO: 1 (N-terminal region of Rib) 
MAEVISGSAVTLNTNMTKNVQNGRAYIDLYDVKNGKIDPLQLITLNSPDLKAQYVIRQGGN
YFTQPSELTTVGAASINYTVLKTDGSPHTKPDGQVDIINVSLTIYNSSALRDKIDEVKKKAE
DPKWDEGSRDKVLISLDDIKTDIDNNPKTQSDIANKITEVTNLEKILVPRIP
SEQ ID NO: 2 (N-terminal region of AlpC) 
STIPGSAATLNTSITKNIQNGNAYIDLYDVKLGKIDPLQLIVLEQGFTAKYVFRQGTKYYGD
VSQLQSTGRASLTYNIFGEDGLPHVKTDGQIDIVSVALTIYDSTTLRDKIEEVRTNANDPK
WTEESRTEVLTGLDTIKTDIDNNPKTQTDIDSKIVEVNELEKLLVLS
SEQ ID NO: 3 (GBS-NN): 
MAEVISGSAVTLNTNMTKNVQNGRAYIDLYDVKNGKIDPLQLITLNSPDLKAQYVIRQGGN
YFTQPSELTTVGAASINYTVLKTDGSPHTKPDGQVDIINVSLTIYNSSALRDKIDEVKKKAE
DPKWDEGSRDKVLISLDDIKTDIDNNPKTQSDIANKITEVTNLEKILVPRIPEFSTIPGSAAT
LNTSITKNIQNGNAYIDLYDVKLGKIDPLQLIVLEQGFTAKYVFRQGTKYYGDVSQLQSTG
RASLTYNIFGEDGLPHVKTDGQIDIVSVALTIYDSTTLRDKIEEVRTNANDPKWTEESRTE
VLTGLDTIKTDIDNNPKTQTDIDSKIVEVNELEKLLVLS
SEQ ID NO: 4 (N-terminal region of Alp1) 
MAEVISGSAATLNSALVKNVSGGKAYIDIYDVKNGKIDPLNLIVLTPSNYSANYYIKQGGRI
FTSVNQLQTPGTATITYNILDENGNPYTKSDGQIDIVSLVTTVYDTTELRNNINKVIENAND
PKWSDDSRKDVLSKIEVIKNDIDNNPKTQSDIDNKIVEVNELEKLLVLP
SEQ ID NO: 5 (N-terminal region of Alp2) 
STIPGSAATLNTSITKNIQNGNAYIDLYDVKNGLIDPQNLIVLNPSSYSANYYIKQGAKYYS
NPSEITTTGSATITFNILDETGNPHKKADGQIDIVSVNLTIYDSTALRNRIDEVINNANDPKW
SDGSRDEVLTGLEKIKKDIDNNPKTQIDIDNKINEVNEIEKLLVVSL
SEQ ID NO: 6 (GBS-NN2, N-terminal Met removed post-translationally): 
MAEVISGSAATLNSALVKNVSGGKAYIDIYDVKNGKIDPLNLIVLTPSNYSANYYIKQGGRI
FTSVNQLQTPGTATITYNILDENGNPYTKSDGQIDIVSLVTTVYDTTELRNNINKVIENAND
PKWSDDSRKDVLSKIEVIKNDIDNNPKTQSDIDNKIVEVNELEKLLVLPEFSTIPGSAATLN
TSITKNIQNGNAYIDLYDVKNGLIDPQNLIVLNPSSYSANYYIKQGAKYYSNPSEITTTGSAT
ITFNILDETGNPHKKADGQIDIVSVNLTIYDSTALRNRIDEVINNANDPKWSDGSRDEVLTG
LEKIKKDIDNNPKTQIDIDNKINEVNEIEKLLVVSL
SEQ ID NO: 7 (N-terminal of Alp4)

VVEGSAATLNTAMTKNMQNGNAYIDIYDVKLGKIDPLQLIKLEPGYTAIYYITQGSKVYA
NVSELQTPGAAKVNYRIQTSDGSDHIKSDGQLDSVNISLTVYD

### EXAMPLES

### Example 1: the vaccine formulation candidates

The target product profile for the development was a liquid vaccine formulation that contained GBS-NN (0.1 mg/ml), GBS-NN2 (0.1 mg/ml) and alhydrogel (1 mg Al³⁺/ml). The vaccine formulation should be stable during long term storage (12-24 months) at 2-8 °C and during short and intermediate term storage (3-9 months) at 25 °C (room temperature). The intended dosage would be 500 µl vaccine formulation providing 50 µg GBS-NN and 50 µg GBS-NN2.

A number of vaccine formulation candidates were formulated according to table 1 below. In addition to the buffer, optional salt and optional sugar as detailed in table 1, each of the vaccine formulation candidate included 0.1 mg/ml of GBS-NN fusion protein and 0.1 mg/ml of GBS-NN2 fusion protein adsorbed to Alhydrogel (AIOH) corresponding to a 1 mg/ml Al³⁺ concentration. Each formulation was made in a volume of 700 µl. General stability testing was performed at 0, 1, 3, 6, 9, 12, 24, and 36 months.

**Table 1: Vaccine formulation candidates**

| **No.** | **Formulation** | **Buffer** | **pH** | **Salt** | **Sugar** |
|---|---|---|---|---|---|
| 1 | F(DP)1 | 2 mM Na/K-phosphate | 7.2 | 150 mM NaCl | - |
| 2 | F(DP)2 | | 7.2 | - | 300 mM sorbitol |
| 3 | F(DP)3 | | | - | 300 mM sucrose |
| 4 | F(DP)4 | 10 mM L-histidine HCl | 6.2 | 150 mM NaCl | - |
| 5 | F(DP)5 | | 6.7 | | - |
| 6 | F(DP)6 | | 6.2 | - | 300 mM sorbitol |
| 7 | F(DP)7 | 10 mM Tris HCl | 7.2 | 150 mM NaCl | - |

The excipients listed in table 2 were used for preparing the vaccine formulation candidates.

**Table 2: Materials**

| **Material** | **Product no.** | **Manufacturer** |
|---|---|---|
| NaCl | 1.16224.5000 | Merck-Millipore |
| Na₂HPO₄ · 2H₂O | 1.06576.1000 | Merck-Millipore |
| KH₂PO₄ | 1.04871.1000 | Merck-Millipore |
| Alhydrogel 2% EP, 10 mg/ml Al | 843161 | Brenntag |
| Tris | 1.08386.1000 | Merck-Millipore |
| D(+)-sucrose | 141621.1211 | PanReac AppliChem |
| L-histidine | 1.04352.1000 | Merck-Millipore |
| L-histidine-HCl | 1.04354.0500 | Merck-Millipore |
| D(-)-sorbitol | APPCA2222.1000 | AppliChem |
| Hydrochloric acid, 1 M | 30024.290 | VWR |
| Sodium hydroxide solution; 1 mol/l | 1.09137.1000 | Merck-Millipore |

The GBS-NN and GBS-NN2 drug substances were provided according to table 3:

**Table 3: GBS-NN and GBS-NN2 drug substances**

| **Material** | **Description** |
|---|---|
| GBS-NN | GMP manufactured; conc 1.75 mg/ml in 7 mM phosphate, 150 mM NaCl pH 7.2 |
| GBS-NN2 | GMP manufactured; conc 1.75 mg/ml in 7 mM phosphate, 150 mM NaCl pH 7.2 |

The vaccine formulation candidates were prepared as follows.

Samples of the drug substances GBS-NN and GBS-NN2 were prepared by dialysis for buffer exchange in ≥ 3 steps each for ≥ 2 h (last step overnight) with in total ≥ 300-fold buffer excess over the sample volume at room temperature (RT). Dialysis was performed by using Slide-A-Lyzer dialysis cassettes (10-kDa membrane). For both GBS-NN DS and GBD-NN2 drug substance, approximately 35 ml of the 1.75 mg/ml solution were transferred into a 25 - 70 ml dialysis cassette per formulation.

The dialysis was performed with formulation buffer according to table 4.

**Table 4: Formulation buffer**

| **No.** | **Formulation** | **Buffer** | **pH** | **Salt** | **Sugar** |
|---|---|---|---|---|---|
| 1 | F(NN)1/ F(NN2)1 | 7 mM Na/K-phosphate | 7.2 | 150 mM NaCl | - |
| 2 | F(NN)2/ F(NN2)2 | 7 mM Na/K-phosphate | 7.2 | - | 300 mM sorbitol |
| 3 | F(NN)3/ F(NN2)3 | | | - | 300 mM sucrose |
| 4 | F(NN)4/ F(NN2)4 | 20 mM L-histidine HCl | 6.2 | 150 mM NaCl | - |
| 5 | F(NN)5/ F(NN2)5 | | 6.7 | | - |
| 6 | F(NN)6/ F(NN2)6 | | 6.2 | - | 300 mM sorbitol |
| 7 | F(NN)7/ F(NN2)7 | 20 mM Tris HCl | 7.2 | 150 mM NaCl | - |

After dialysis, the GBS-NN and GBD-NN2 samples, which were dialyzed against the same formulation buffer, were used for the preparation of the seven vaccine formulations with a final target composition of 0.1 mg/ml GBS-NN, 0.1 mg/ml GBS-NN2 and 1 mg/ml Alhydrogel (Al³⁺).

Alhydrogel 2% EP, 10 mg/ml Al³⁺ contains no further excipients. To have maximum flexibility in excipient selection, the Alhydrogel was not transferred or diluted into any other buffer.

A generalized pipetting scheme was used for each formulation preparation (exemplary for the drug substance formulations at 1 mg/ml in table 5 and table 6).

**Table 5: Generalized pipetting scheme**

| **Component** | **Volume [ml)** |
|---|---|
| Alhydrogel (10 mg/ml Al³⁺) | 10 |
| Dilution buffer (DB) | 70 |
| GBS-NN (1 mg/ml) | 10* |
| GBS-NN2 (1 mg/ml) | 10* |
| Vaccine formulation (drug product) | 100 |

| | |
|---|---|
| *premixed before adding to Alhydrogel/dilution buffer mix. | |

**Table 6: Dilution buffer compositions**

| **Dilution buffer** | **Excipient** | **Excipient concentration [mM]** |
|---|---|---|
| DB-1 | Na/K phosphate | 0.86 |
| | NaCl | 171 |
| DB-2 | Na/K phosphate | 0.86 |
| | Sorbitol | 343 |
| DB-3 | Na/K phosphate | 0.86 |
| | Sucrose | 343 |
| DB-4 | His HCl pH 6.2 | 8.57 |
| | NaCl | 171 |
| DB-5 | His HCl pH 6.7 | 8.57 |
| | NaCl | 171 |
| DB-6 | His HCl pH 6.2 | 8.57 |
| | Sorbitol | 343 |
| DB-7 | Tris HCl | 8.57 |
| | NaCl | 171 |

For mixing all components of the vaccine formulations (total volume 110 ml), a 22-ml mixture of 0.5 mg/ml GBS-NN and 0.5 mg/ml GBS-NN2 was prepared with the two corresponding dialyzed and sterile filtered drug substance materials (and the corresponding sterile filtered formulation buffer to dilute, if necessary). Twenty ml of this mixture was added to the 80 ml Alhydrogel mixture (10 ml of 10 mg/ml alhydrogel + 70 ml sterile filtered corresponding dilution buffer).

After preparation, the vaccine formulations were homogenized by rotating on a vertical wheel at 10 rpm for 10 minutes. Vaccine formulations were stored at 2-8 °C and rehomogenized before final filling onto container.

pH was measured with a calibrated pH meter (SevenCompact, Mettler Toledo AG) by using a normal ionic strength (> 100 mM) electrode (InLab Micro Pro-ISM). For the measurement, 200 µl of undiluted sample were transferred to a 1.5-ml Eppendorf tube. The electrode was cleaned with highly purified water.

The final concentration of the components in formulation F(DP)1 is given by table 7.

**Table 7: Composition of formulation F(DP)1**

| Component | Concentration [mg/ml] |
|---|---|
| GBS-NN | 0.100 |
| GBS-NN2 | 0.100 |
| Al3+ as alhydrogel | 1.000 |
| Na₂HPO₄ · 2H₂O | 0.267 |
| KH₂PO₄ | 0.068 |
| NaCl | 8.766 |

The weight ratio of Na₂HPO₄ · 2H₂O to KH₂PO₄ in the Na/K-phosphate buffer was 3.92:1. Based on table 7 and the molecular weights of Na₂HPO₄ · 2H₂O and KH₂PO₄ (177.99 g/mol and 136.086 g/mol, respectively), 1.5 mM of the 2 mM concentration of phosphate in the Na/K phosphate buffer was from the Na₂HPO₄ · 2H₂O and the remaining 0.5 mM concentration of phosphate was from the KH₂PO₄.

Accordingly, the molar ratio of Na₂HPO₄ · 2H₂O to KH₂PO₄ in the Na/K-phosphate buffer was 3:1.

### Example 2: Desorption of fusion proteins from the candidate vaccine formulations after storage

In this example the desorption of the fusion proteins from the vaccine formulation candidates was investigated using reversed-phase high performance liquid chromatography (RPHPLC). Samples were swirled in order to resuspend the Alhydrogel. 100 µl of this resuspended sample was transferred to an Eppendorf (Protein LoBind) tube and spun in a tabletop centrifuge for 5 min. The supernatant was analyzed.

The following parameters were used for the RP-HPLC analysis:
Instrument: Ultimate 3000 (Dionex) or HP1100 (Agilent Technology)
Column: Aeris^{™} 3.6 µm WIDEPORE C4 200 Å, LC Column 150 x 4.6 mm
Security Guard: SecurityGuard^{™} ULTRA Cartridges UHPLC WIDEPORE C4 4.6mm ID
Flow rate: 1.0 ml/min
Mobile phases: Mobile phase A: 0.1% TFA, 15% isopropanol (IPA) in water
Mobile phase B: 0.1% TFA, 15% IPA in acetonitrile (ACN)
Detection: UV at 214 nm and 280 nm
Column oven: 40 °C
Sample cooling: 8 ± 4 °C
Injection volume: 10 µl
Analysis time: 38 min

The desorption was measured as the concentration of free fusion protein after various lengths of storage at various temperatures.

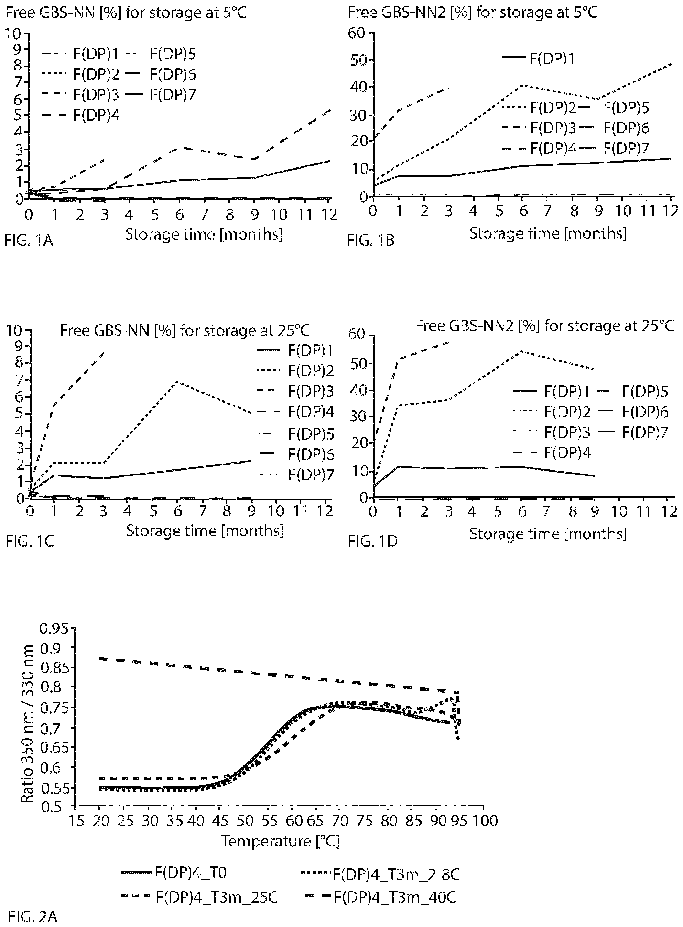

As shown in Fig. 1A-1D, 100% of both proteins remain bound over time in the formulations F(DP)4, F(DP)5, F(DP)6 and F(DP)7 that did not include phosphate. For the remaining formulations F(DP)1, F(DP)2, and F(DP)3, the formulation with NaCl at 150mM, i.e. formulation F(DP)1, had the lowest desorption.

As an immediate result, it was noted that formulation F(DP)3 had an unacceptable high extent of desorption.

Generally, desorption of fusion proteins plateaus after 3-6 months at 2-8°C and after 1 month at 25°C. After 9 months at 25°C desorption is no higher than at 2-8°C.

### Example 3: Thermostability of fusion proteins in candidate vaccine formulations

In this example the thermostability of the fusion proteins in the candidate vaccine formulations was investigated by means of nano Differential Scanning Fluorimetry, nanoDSF. This method uses the intrinsic fluorescence of the protein and was performed with a Prometheus NT.48 system, featuring back reflection optics (NanoTemper Technologies).

The intrinsic fluorescence of tyrosine and tryptophan residues was monitored upon heating. Unfolding of the first and second fusion proteins exposes the aromatic amino acids to the aqueous environment, leading to a shift of the emission maximum towards higher wavelength. Consequently, an increase in the ratio of the fluorescence intensities monitored at 350 and 330 nm was observed upon heating. For each respective vaccine candidate formulations F(DP)1 - F(DP)7, three high-sensitivity glass capillaries (NanoTemper Technologies) were loaded with the undiluted test samples (n=3; less than 50 µl per triplicate required). The excitation power was adapted to meet a suitable fluorescence signal range with the respective sample concentration. The samples were heated from 20 to 95 °C with a scan rate of 1 °C/min. Apparent protein melting temperatures (Tm) were obtained from the ratio of the fluorescence intensity at 350 nm and 330 nm (ratio 350 nm/330 nm) as function of temperature as well as the fluorescence intensity at 350 nm and 330 nm, respectively. To that end, the first apparent melting temperature Tm1 was determined from the fluorescence intensity at 330 nm or 350 nm, depending on which wavelength showed the first transition clearer, and Tm2 from the ratio 350 nm/330 nm. The Tm values, and the onset temperature of unfolding (Tm,onset), based on the ratio 350 nm/330 nm, were calculated by the PR.Control software v2.1.1 (NanoTemper Technologies). In cases of less pronounced unfolding transitions, the inflection points were manually determined based on local maxima of the 1st derivative curves.

The sample of the respective vaccine candidate formulations F(DP)1 - F(DP)7 had been stored for 3 months at 2-8 °C, 25°C or 40°C, or had not been stored. According to the experiment, an ideal vaccine formulation candidate would be expected to show a similar curve of gradual unfolding during heating regardless of the storage conditions of the vaccine formulations as this would indicate that the vaccine formulation was capable of preserving the folding of the fusion proteins regardless of storage conditions.

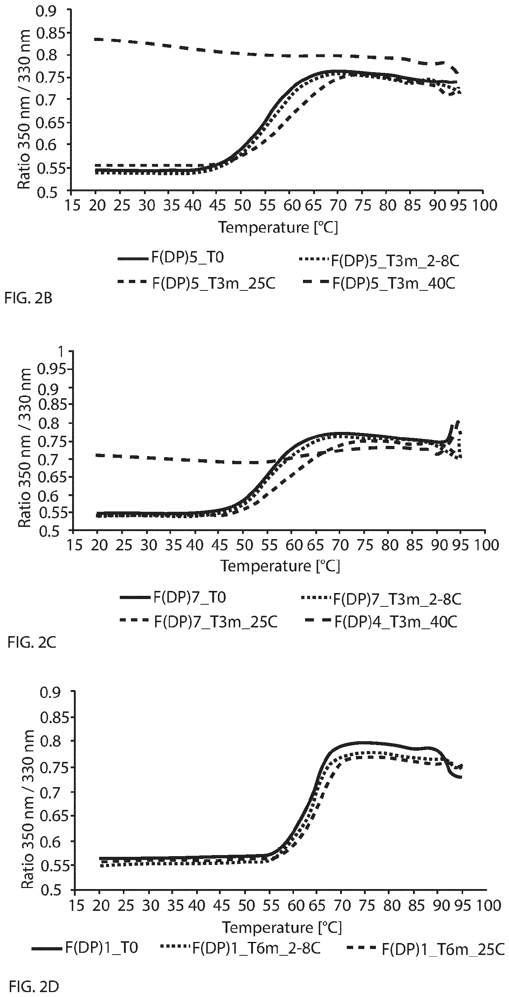

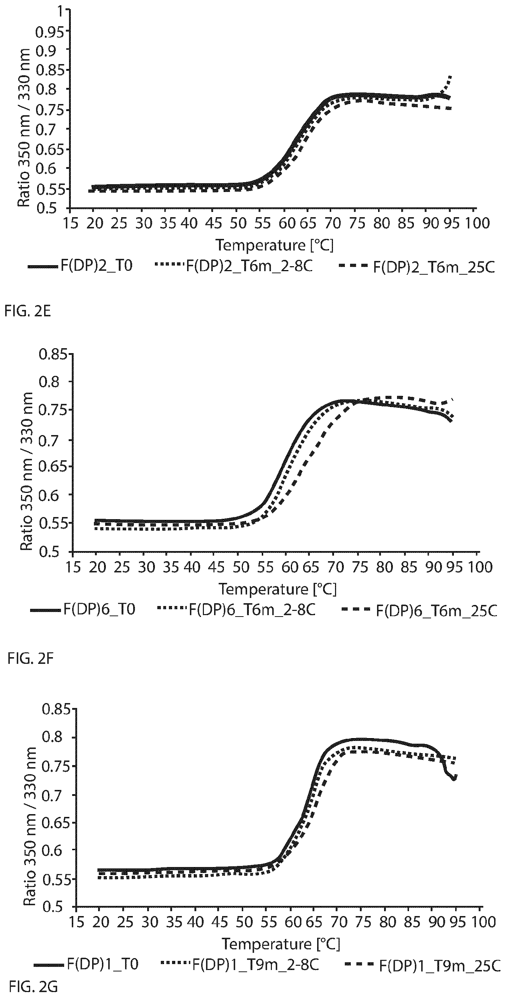

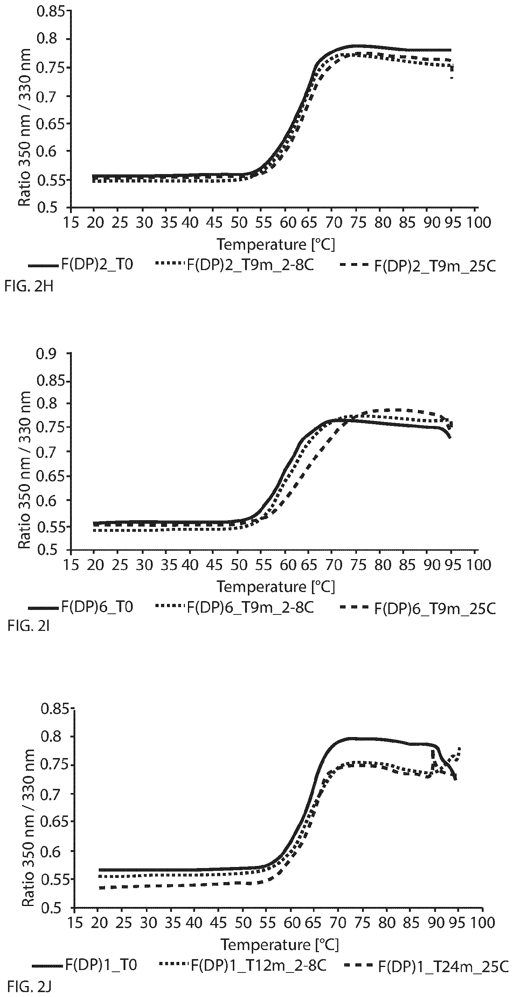

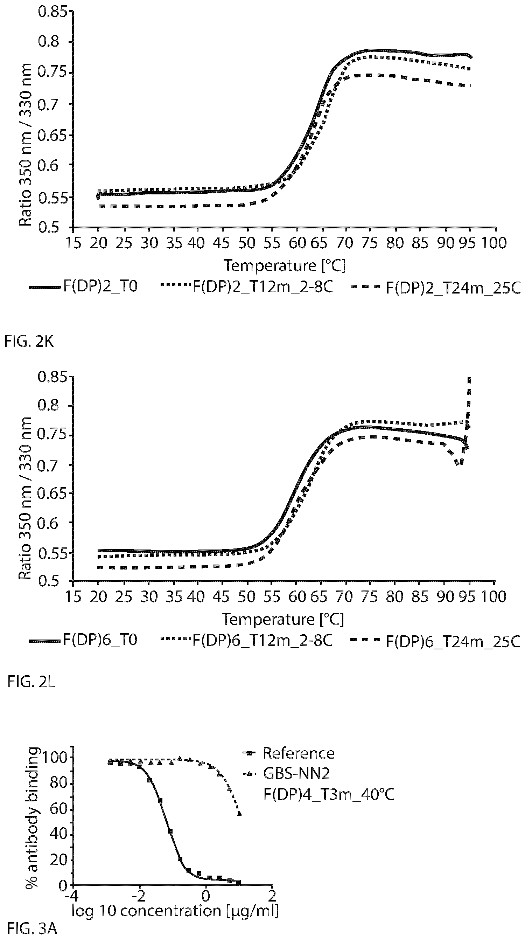

Analysis of the curves, see the selected curves in figs. 2A-2L, suggests two transition temperatures which are interpreted as representing the first fusion protein (GBS-NN) and the second fusion protein (GBS-NN2).

As seen in figs. 2A-2C, for vaccine formulation candidates F(DP)4, F(DP)5 and F(DP)7, i.e. the formulations with 10 mM L-histidine HCl or 10 mM Tris HCl, there is almost a complete unfolding of the fusion proteins already after 3 months at 40°C as evidence by the largely straight line which shows that the fusion proteins were already unfolded at the start of the nano differential scanning fluorimetry experiment.

Further, these vaccine formulation candidates also had a partial unfolding already at the start of the nano differential scanning fluorimetry experiment when they had been stored for 3 months at 25°C, as can be seen by the curve for T3m_25C not following the curves for the non-stored samples (4_T0) and the samples stored for 3 months at 2-8°C. Accordingly, figs 2A-C showed that vaccine formulation candidates F(DP)4, F(DP)5 and F(DP)7 did not provide a suitable stability to the fusion proteins as the fusion proteins in these vaccine formulation candidates had started to partially unfold already after 3 months of storage at 25°C. In other words, these vaccine formulation candidates would require refrigerated storage at 2-8°C if they were to preserve the fusion proteins in the intended folded state for 3 months of storage. As such storage requirements are disadvantageous, vaccine formulation candidates F(DP)4, F(DP)5 and F(DP)7 were deselected from further study.

As seen in figs. 2D-2F, for vaccine formulation candidates F(DP)1, F(DP)2 and F(DP)6 after no or 6 months storage at 2-8°C or 25°C, these vaccine formulation candidates, which all include phosphate, provided much better stability to the fusion proteins than the F(DP)4, F(DP)5 and F(DP)7 formulations. Specifically, as seen in figs 2D-2F, the curves for 25°C storage for 6 months (T6m_25C) are very similar to the curves for the storage at 2-8°C (T6m_2-8C) and the curves for the non-stored samples (T0) for vaccine formulation candidates F(DP)1 and F(DP)2 whereas the corresponding curves for vaccine formulation candidate F(DP)6 show a later onset in fluorescence increase indicating that the fusion proteins in the F(DP)6 vaccine formulation were more stable against the heat induced unfolding during the experiment after having been stored at 25°C compared to having been stored at the lower temperature 2-8°C or not stored at all.

Figs. 2G-2I thus show further results obtained for longer storage (9 months) of samples of vaccine formulation candidates F(DP)1, F(DP)2 and F(DP)6. Similar to figs. 2D-2F, the curves for 25°C storage for 9 months (T9m_25C) are very similar to the curves for the storage at 2-8°C (T9m_2-8C) and the curves for the non-stored samples (T0) for vaccine formulation candidates F(DP)1 and F(DP)2 whereas the corresponding curves for vaccine formulation candidate F(DP)6 show a later onset in fluorescence increase indicating that the fusion proteins in the F(DP)6 vaccine formulation were more stable against the heat induced unfolding during the experiment after having been stored at 25°C compared to having been stored at the lower temperature 2-8°C or not stored at all.

Finally, figs 2J-2L show results obtained for 12 and 24 months storage at a typical storage temperature of 5°C. As seen from the curves, there is no significant difference between 12 and 24 months storage for F(DP)1, F(DP)2 and F(DP)6.

Accordingly, Example 3 showed that vaccine formulation candidates F(DP)1 and F(DP)2, closely followed by F(DP)6, provided the least change of folding of the fusion proteins during storage.

The following table 8 shows the summarized results of example 3:

**Table 8: Summarized results from example 3**

| **Formulation** | **Tₘ₂ [°C]** | | | | | |
|---|---|---|---|---|---|---|
| | **T0** | **T3m** | | | **T6m** | |
| | | **2-8 °C** | **25 °C** | **40 °C** | **2-8 °C** | **25 °C** |
| F(DP)1 | 64.5 ± 0.0 | 64.5 ± 0.1 | 65.3 ± 0.0 | 66.9 ± 0.0 | 64.7 ± 0.0 | 65.8 ± 0.0 |
| F(DP)2 | 63.9 ± 0.0 | 63.6 ± 0.0 | 63.9 ± 0.0 | 65.1 ± 0.0 | 63.6 ± 0.0 | 64.3 ± 0.0 |
| F(DP)3 | 64.1 ± 0.1 | 63.8 ± 0.1 | 64.4 ± 0.1 | 65.0 ± 0.1 | | |
| F(DP)4 | 54.7 ± 0.2 | 55.3 ± 0.1 | 60.7 ± 0.3 | n.d. | | |
| F(DP)5 | 55.0 ± 0.0 | 55.7 ± 0.2 | 60.8 ± 0.6 | n.d. | | |
| F(DP)6 | 59.9 ± 0.2 | 60.6 ± 0.1 | 63.1 ± 0.3 | 68.1 ± 0.1 | 60.7 ± 0.1 | 63.9 ± 0.0 |
| F(DP)7 | 54.9 ± 0.1 | 55.4 ± 0.1 | 59.7 ± 0.4 | 64.6 ± 0.2 | | |

| **Formulation** | **T9m** | | **T12m** | | **T24m** | |
|---|---|---|---|---|---|---|
| | **2-8 °C** | **25 °C** | **2-8 °C** | | **2-8 °C** | |
| F(DP)1 | 64.6 ± 0.0 | 66.1 ± 0.1 | 64.8 ± 0.0 | | 64.7 ± 0.2 | |
| F(DP)2 | 63.4 ± 0.0 | 64.1 ± 0.2 | 62.1 ± 0.1 | | 63.2 ± 0.0 | |
| F(DP)6 | 60.7 ± 0.0 | 64.6 ± 0.3 | 60.9 ± 0.2 | | 59.8 ± 0.0 | |

The parameter Tₘ₂ indicates the temperature where 50% of maximum increase in fluorescence is observed and was determined from the ratio F350 nm/F330 nm of nanoDSF measurements, and Tₘ₂ was also used as the measure of the thermal stability.

Summarizing Example 2 and Example 3, the overall best vaccine formulation candidate was F(DP)1 followed by F(DP)6, where the latter had less desorption than F(DP)1 but exhibited nano differential scanning fluorimetry intensity curves for storage at 25°C that differed more from the curves obtained for storage at lower temperatures. F(DP)1 also had a higher Tm2 than F(DP)6 for all storage temperatures and storage times except 3 months at 40°C.

### Example 4: Potency assay of vaccine formulation candidates

The potency of the GBS-NMN antigen and the GBS-NN2 antigen when 1 formulated in the various vaccine formulation candidates was tested in an inhibition ELISA. The reference was a preparation of the respective GBS-NN and GBS-NN2 fusion proteins that had been freshly prepared and not stored. The ELISA used antibodies against the respective fusion protein and the % of antibody binding to the respective fusion protein was determined for various concentrations of the fusion proteins, thereby showing whether or not the fusion proteins, in the respective vaccine formulations candidates, bound the respective antibody to the same extent at the same concentration as the reference fusion protein despite being present in the respective buffer of the respective vaccine formulation candidate and having been stored at the various temperatures for the various times.

The Inhibition assay was performed on the most promising candidates F(DP)1, F(DP)2 and F(DP)6, as well as the less promising candidates F(DP) 3 and F(DP)4.

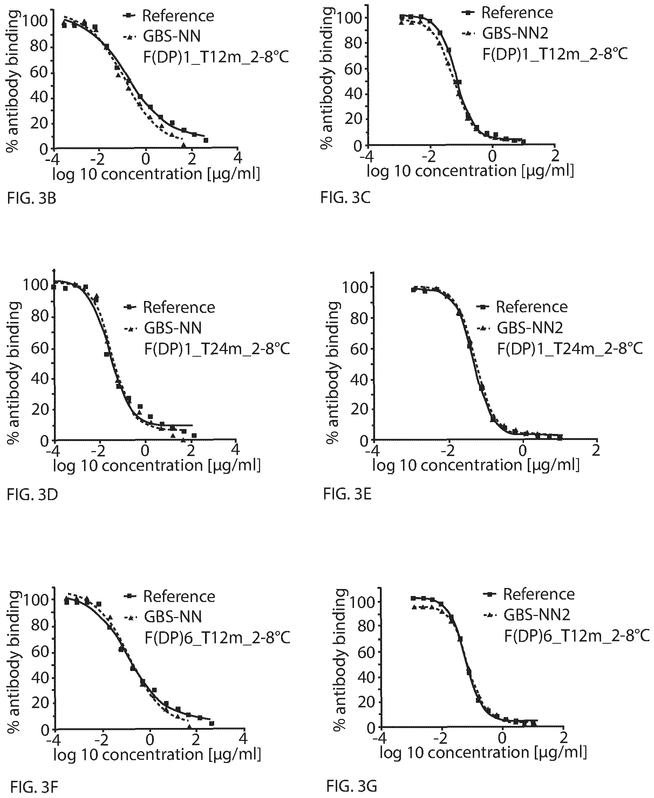

The results are shown in figs 3A-3G and in table 9 below.

**Table 9: Results of potency assay**

| **Sample** | **GBS-NN** | | **GBS-NN2** | |
|---|---|---|---|---|
| | **Ratio potency [%]** | **95% confidence interval [%]** | **Ratio potency [%]** | **95% confidence interval [%]** |
| F(DP)3_T3m_40°C | 61 | 22-169 | 109 | 99-120 |
| F(DP)4_T3m_40°C | 1; not parallel | - | <1 | - |
| F(DP)1_T6m_2-8°C | 77 | 28-215 | 89 | 82-97 |
| F(DP)1_T6m_25°C | 91 | 34-245 | 93 | 84-104 |
| F(DP)2_T6m_2-8°C | 70 | 30-163 | 97 | 88-107 |
| F(DP)2_T6m_25°C | 83 | 38-180 | 83 | 77-90 |
| F(DP)6_T6m_2-8°C | 61 | 17-212 | 74 | 69-80 |
| F(DP)6_T6m_25°C | 59 | 24-141 | 63 | 59-67 |
| F(DP)1_T12m_2-8°C | 144 | 61-341 | 121 | 108-136 |
| F(DP)6_T12m_2-8°C | 86 | 41-182 | 83 | 76-91 |
| F(DP)1_T24m_2-8°C | 76 | 41-139 | 92 | 85-99 |

As seen from table 8 and fig. 3A, the F(DP)3 and F(DP)4 vaccine formulation candidates performed poorly with a large difference between the inhibition curve compared to the reference, as well as a low ratio of potency compared to the reference. Vaccine formulation candidate F(DP)1 and F(DP)6 however provided inhibition curves that were matched the curves for the reference well despite storage of up to 12 and 24 months at 2-8 °C and with high potency ratios for both the fusion proteins GBS-NN and GBS-NN2. Generally, the F(DP)1 vaccine formulation candidate provided the best results.

### Example 5 Summarization of results

Table 10 below shows the summarized evaluation of the vaccine formulation candidates.

**Table 10. Summarized evaluation of the vaccine formulation candidates**

| Formulation | content | verdict |
|---|---|---|
| F(DP)1 | 2 mM Na/K-phosphate, 150 mM NaCL, pH 7.2 | Best formulation |
| F(DP)2 | 2 mM Na/K-phosphate, 300 mM sorbitol, pH 7.2 | Deselected due to high desorption rate at 25°C |
| F(DP)3 | 2 mM Na/K-phosphate, 300 mM sucrose, pH 7.2 | Deselected due to low initial adsorption |
| F(DP)4 | 10 mM L-histidine HCL, 150 mM NaCl, pH 6.2 | Deselected due to thermal instability at 40°C after 3 months, partial unfolding seen at 25°C |
| F(DP)5 | 10 mM L-histidine HCL, 150 mM NaCl, pH 6.7 | Deselected due to thermal instability at 40°C after 3 months, partial unfolding seen at 25°C |
| F(DP)6 | 10 mM L-histidine HCL, 300 mM sorbitol, pH 6.2 | Similar performance to F(DP)1. |
| F(DP)7 | 10 mM Tris HCL, 150 mM NaCl, pH 7.2 | Deselected due to thermal instability at 40°C after 3 months, partial unfolding seen at 25°C |

## Claims

1. A vaccine formulation comprising:
a) an antigen comprising an amino acid sequence having at least 90% sequence identity with the amino acid sequence of the N-terminal region of a group B *Streptococcus* surface protein, and
b) a buffer having a pH of 6 to 8 and comprising:
a. aluminum hydroxide gel particles, and
b. a buffer system comprising:
i. phosphate, and optionally also a salt, or
ii. histidine, and optionally also a sugar.

2. The vaccine formulation according to claim 1, wherein the antigen comprises a fusion protein comprising or consisting of a first amino acid sequence having at least 90% sequence identity with the amino acid sequence of the N-terminal region of a first group B *Streptococcus* surface protein, said first amino acid sequence being fused, optionally via linker, to a second amino acid sequence having at least 90% sequence identity with the amino acid sequence of the N-terminal region of a second group B *Streptococcus* surface protein.

3. The vaccine formulation according to claim 2, wherein the first group B *Streptococcus* surface protein is selected from the group consisting of Rib protein and Alp1 protein, and wherein the second group B *Streptococcus* surface protein is selected from the group consisting of AlpC protein, Alp2 protein, Alp3 protein, and Alp4 protein.

4. The vaccine formulation according to claim 3, wherein the first group B *Streptococcus* surface protein is Rib protein, and wherein the second group B *Streptococcus* surface protein is AlpC protein.

5. The vaccine formulation according to claim 4, wherein the fusion protein consists of:
- a first amino acid sequence part consisting of 170 to 178 amino acids, preferably 174 to 175 amino acids, and having at least 90% sequence identity with the amino acid sequence shown in SEQ ID NO: 1;
- a second amino acid sequence part consisting of 165 to 174 amino acids, preferably 169 to 170 amino acids, and having at least 90% sequence identity with the amino acid sequence shown in SEQ ID NO: 2; and optionally:
- a linker amino acid sequence part consisting of 1 to 20 amino acids and separating the first amino acid sequence part from the second amino acid part,
and wherein the first fusion protein preferably consists of 335 to 372 amino acids, preferably 343 to 353 amino acids, more preferably 343 to 347 amino acids,
and wherein preferably the first fusion protein has an amino acid sequence having at least 90%, at least 92%, at least 94%, at least 96%, at least 98%, at least 99%, or 100% identity to SEQ ID NO: 3.

6. The vaccine formulation according to claim 3, wherein the first group B *Streptococcus* surface protein is Alp1 protein, and wherein the second group B *Streptococcus* surface protein is Alp2 protein.

7. The vaccine formulation according to claim 6, wherein the fusion protein consists of:
- a first amino acid sequence part consisting of 168 to 177 amino acids, preferably 172 to 173 amino acids, and having at least 90% sequence identity with the amino acid sequence shown in SEQ ID NO: 4,
- a second amino acid sequence part consisting of 168 to 178 amino acids, preferably 172 to 173 amino acids, and having at least 90% sequence identity with the amino acid sequence shown in SEQ ID NO: 5, and optionally,
- a linker amino acid sequence part consisting of 1 to 20 amino acids and separating the first amino acid sequence part from the second amino acid part, and wherein the second fusion protein preferably consists of 335 to 372 amino acids, preferably 343 to 353 amino acids, more preferably 343 to 347 amino acids, and wherein preferably the second fusion protein has an amino acid sequence having at least 90%, at least 92%, at least 94%, at least 96%, at least 98%, at least 99%, or 100% identity to SEQ ID NO: 6.

8. The vaccine formulation according to any preceding claim, wherein the antigen comprises or consists of:
- a first fusion protein as defined in claim 4 or 5, preferably as defined in claim 5, and
- a second fusion protein as defined in claim 6 or 7, preferably as defined in claim 7.

9. The vaccine formulation according to claim 8, wherein:
- the concentration of the first fusion protein, in the vaccine formulation, is from 0.05 to 0.15 mg/ml, preferably 0.75 to 0.125 mg/ml, more preferably 0.09 to 0.11 mg/ml, such as 0.1, mg/ml, and
- the concentration of the second fusion protein, in the vaccine formulation, is from 0.05 to 0.15 mg/ml, preferably 0.75 to 0.125 mg/ml, more preferably 0.09 to 0.11 mg/ml, such as 0.1 mg/ml.

10. The vaccine formulation according to any preceding claim, wherein the buffer comprises 0.5 - 1.5 mg/ml, preferably 0.75-1.25 mg/ml, more preferably 0.9-1.1. mg/ml, such as 1 mg/ml, of aluminum hydroxide gel particles.

11. The vaccine formulation according to any preceding claim, wherein the buffer system comprises:
i. 1-3 mM, preferably 1.5 to 2.5 mM, more preferably 2 mM phosphate, and preferably also 100-200 mM, preferably 125-175 mM, more preferably 150 mM NaCl, or
ii. 5-15, preferably 8-12, more preferably 10 mM histidine, and preferably also 200-400 mM, preferably 250-350 mM, more preferably 300 mM sorbitol.

12. The vaccine formulation according to any preceding claim, comprising.
a) an antigen consisting of 0.1 mg/ml of a first fusion protein having the amino acid sequence of SEQ ID NO: 3 and 0.1 mg/ml of a second fusion protein having the amino acid sequence of SEQ ID NO: 6, and
b) a buffer having a pH of 7.2 and comprising:
a. 1 mg/ml aluminum hydroxide gel particles,
b. 2 mM Na/K phosphate, and
c. 150 mM NaCl.

13. A method of producing a vaccine formulation comprising the steps of:
i. providing an antigen comprising an amino acid sequence having at least 90% sequence identity with the amino acid sequence of the N-terminal region of a group B *Streptococcus* surface protein,
ii. providing a buffer having a pH of 6 to 8 and comprising:
a. aluminum hydroxide gel particles, and
b. a buffer system comprising:
i. phosphate, and optionally also a salt, or
ii. histidine, and optionally also a sugar, and
iii. mixing the antigen with the buffer.

14. The vaccine formulation according to any of the claims 1-12 for use in a method of a reducing, preventing and/or treating a GBS infection.

15. The vaccine formulation for use according to claim 14, wherein the vaccine formulation is administered to a subject, wherein the subject preferably is a female subject, wherein the subject preferably is a female subject carrying an unborn offspring.
